(19) Europäisches Patentamt
European Patent Office
Office européen des brevets

(11) EP 4 570 249 A1

(12) **EUROPEAN PATENT APPLICATION**
published in accordance with Art. 153(4) EPC

(43) Date of publication:
**18.06.2025 Bulletin 2025/25**

(21) Application number: **23851630.6**

(22) Date of filing: **31.07.2023**

(51) International Patent Classification (IPC):
***A61K 31/4985*** *(2006.01)* ***C07D 471/04*** *(2006.01)*
***A61P 35/00*** *(2006.01)*

(52) Cooperative Patent Classification (CPC):
**A61K 31/4985; A61P 35/00; C07D 471/04**

(86) International application number:
**PCT/CN2023/110230**

(87) International publication number:
**WO 2024/032409 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.08.2022 CN 202210949131**

(71) Applicant: **Kangbaida (Sichuan) Biotechnology Co., Ltd.**
**Chengdu, Sichuan 610000 (CN)**

(72) Inventors:
- **ZHU, Yuqin**
  **Chengdu, Sichuan 610000 (CN)**
- **WEI, Yonggang**
  **Chengdu, Sichuan 610000 (CN)**
- **CHU, Hongzhu**
  **Chengdu, Sichuan 610000 (CN)**
- **YE, Fei**
  **Chengdu, Sichuan 610000 (CN)**
- **SU, Guizhuan**
  **Chengdu, Sichuan 610000 (CN)**
- **SUN, Yi**
  **Chengdu, Sichuan 610000 (CN)**

(74) Representative: **Kador & Partner Part mbB**
**Corneliusstraße 15**
**80469 München (DE)**

# (54) USE OF PIPERAZINE COMPOUND IN COMBINATION WITH RADIOTHERAPY FOR TREATMENT OF TUMOR

(57) The present application relates to the use of a piperazine compound in combination with radiotherapy for the treatment of tumors.

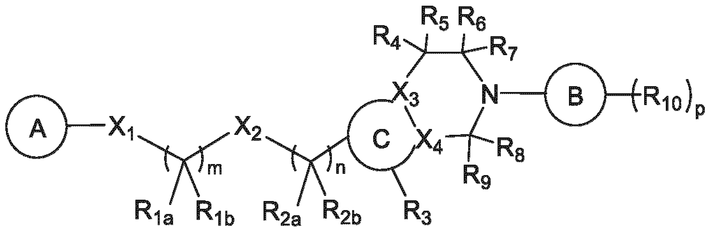

(I)

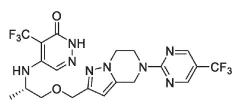

Figure 1

EP 4 570 249 A1

## Description

Technical Field

The present application relates to the use of a piperazine compound in combination with radiotherapy for the treatment of tumors.

Background Art

Radiotherapy is one of the important methods for the comprehensive treatment of malignant tumors, and the neoadjuvant therapy based on conventional long-term radiotherapy has become a standard regimen for treating various malignant tumors. However, long-term clinical studies have found that the responses of patients with malignant tumors to radiotherapy vary greatly. Although some patients can achieve complete pathological remission at the end of a treatment cycle, there are still some patients who cannot benefit from long-term treatment, and a few even develop worsening progress. Therefore, it is clinically significant to improve the therapeutic effect of tumors by combining small molecule anti-tumor compounds and radiotherapy.

Summary

The PCT Application PCT/CN 2022/094124 by the present applicant describes the use of a piperazine derivative in the preparation of a medicament for the treatment of cancer, wherein the compounds described in the specification have a high selectivity and significant inhibitory activity against PARP7. In subsequent studies, it was unexpectedly found that the piperazine derivative can significantly improve the anti-tumor effect of radiotherapy.

Accordingly, the present invention aims to provide the use of a piperazine compound in combination with radiotherapy for the treatment of tumors.

One or more embodiments of the present invention provide the use of a compound of general formula (I), or a pharmaceutically acceptable salt, a stereoisomer or a deuterated compound thereof as an active ingredient in combination with radiotherapy for the treatment of tumors:

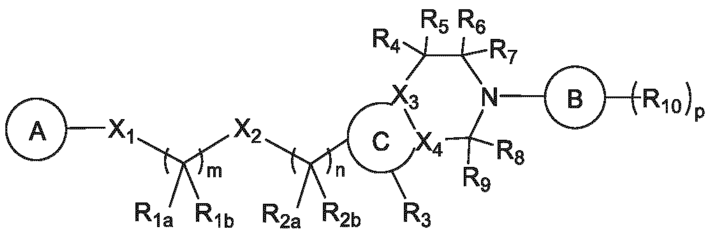

(I)

wherein

$X_1$ is NH, O or 4- to 6-membered heterocycle containing 1 to 3 heteroatoms selected from N and O;
$X_2$ is O or a single bond;
$X_3$ and $X_4$ are each independently C or N;
$R_{1a}$ and $R_{1b}$ are each independently H, D or $C_{1-6}$ alkyl; or $R_{1a}$ and $R_{1b}$ together with the carbon atom connected thereto form 3- to 5-membered cycloalkyl;
$R_{2a}$ and $R_{2b}$ are each independently H, D or $C_{1-6}$ alkyl; or $R_{2a}$ and $R_{2b}$ together with the carbon atom connected thereto form 3- to 5-membered cycloalkyl;
$R_3$ is H, D, $C_{1-6}$ alkyl, halogen or cyano, wherein the $C_{1-6}$ alkyl is optionally substituted with 1 to 3 halogens;
$R_4$ and $R_5$ are each independently H, D or $C_{1-6}$ alkyl; or $R_4$ and $R_5$ together with the carbon atom connected thereto form 3- to 5-membered cycloalkyl;
$R_6$ and $R_7$ are each independently H, D or $C_{1-6}$ alkyl; or $R_6$ and $R_7$ together with the carbon atom connected thereto form C=O;
$R_8$ and $R_9$ are each independently H, D or $C_{1-6}$ alkyl; or $R_8$ and $R_9$ together with the carbon atom connected thereto form C=O; or $R_8$ and $R_9$ together with the carbon atom connected thereto form 3- to 5-membered cycloalkyl;
each $R_{10}$ is independently $C_{1-6}$ alkyl, $C_{1-6}$ alkoxy, $CONR_{10a}R_{10b}$, halogen, cyano, $S(O)_2R_{10c}$, $SR_{10d}$ or 3-to 5-membered cycloalkyl, wherein the $C_{1-6}$ alkyl and $C_{1-6}$ alkoxy are optionally substituted with 1 to 3 halogens;
$R_{10a}$, $R_{10b}$, $R_{10c}$ and $R_{10d}$ are each independently H, D or $C_{1-6}$ alkyl;

A is

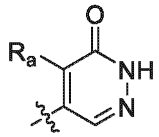

,

and $R_a$ is $C_{1-6}$ alkyl, $C_{3-5}$ cycloalkyl, halogen or cyano, wherein the $C_{1-6}$ alkyl is optionally substituted with 1 to 3 halogens;
B is 5- to 10-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3;
n is 0, 1, 2 or 3; and
p is 0, 1, 2 or 3.

One or more embodiments of the present invention provide the use of a compound of general formula (I), or a pharmaceutically acceptable salt, a stereoisomer or a deuterated compound thereof as an active ingredient in combination with radiotherapy for the treatment of tumors, wherein

$X_1$ is NH, O or 4- to 6-membered heterocycle containing 1 to 3 heteroatoms selected from N and O;
$X_2$ is O or a single bond;
$X_3$ and $X_4$ are each independently C or N;
$R_{1a}$ and $R_{1b}$ are each independently H, D or $C_{1-6}$ alkyl;
$R_{2a}$ and $R_{2b}$ are each independently H, D or $C_{1-6}$ alkyl; or $R_{2a}$ and $R_{2b}$ together with the carbon atom connected thereto form 3- to 5-membered cycloalkyl;
$R_3$ is H, D, $C_{1-6}$ alkyl, halogen or cyano, wherein the $C_{1-6}$ alkyl is optionally substituted with 1 to 3 halogens;
$R_4$ and $R_5$ are each independently H, D or $C_{1-6}$ alkyl; or $R_4$ and $R_5$ together with the carbon atom connected thereto form 3- to 5-membered cycloalkyl;
$R_6$ and $R_7$ are each independently H, D or $C_{1-6}$ alkyl;
$R_8$ and $R_9$ are each independently H, D or $C_{1-6}$ alkyl; or $R_8$ and $R_9$ together with the carbon atom connected thereto form C=O;
each $R_{10}$ is independently $C_{1-6}$ alkyl, $C_{1-6}$ alkoxy, $CONR_{10a}R_{10b}$, halogen, cyano, $S(O)_2R_{10c}$, $SR_{10d}$ or 3-to 5-membered cycloalkyl, wherein the $C_{1-6}$ alkyl and $C_{1-6}$ alkoxy are optionally substituted with 1 to 3 halogens;
$R_{10a}$, $R_{10b}$, $R_{10c}$ and $R_{10d}$ are each independently H, D or $C_{1-6}$ alkyl;
A is

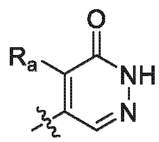

,

and $R_a$ is $C_{1-6}$ alkyl, $C_{3-5}$ cycloalkyl, halogen or cyano, wherein the $C_{1-6}$ alkyl is optionally substituted with 1 to 3 halogens;
B is 5- to 6-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3;
n is 0, 1, 2 or 3; and
p is 0, 1, 2 or 3.

One or more embodiments of the present invention provide the use of a compound of general formula (I-1), or a pharmaceutically acceptable salt, a stereoisomer or a deuterated compound thereof as an active ingredient in combination with radiotherapy for the treatment of tumors:

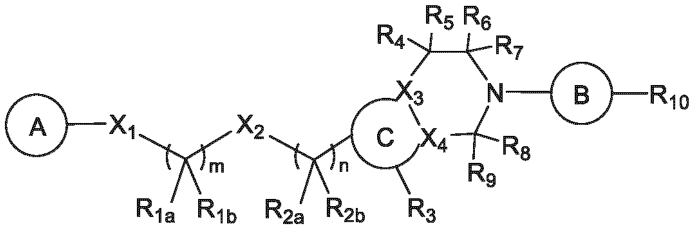

(I-1)

wherein

$X_1$ is NH or 4- to 6-membered heterocycle containing 1 to 3 heteroatoms selected from N and O;
$X_2$ is O;
$X_3$ and $X_4$ are each independently C or N;
$R_{1a}$ and $R_{1b}$ are each independently H, D or $C_{1-6}$ alkyl;
$R_{2a}$ and $R_{2b}$ are each independently H, D or $C_{1-6}$ alkyl;
$R_3$ is H, D, $C_{1-6}$ alkyl or halogen, wherein the $C_{1-6}$ alkyl is optionally substituted with 1 to 3 halogens;
$R_4$ and $R_5$ are each independently H, D or $C_{1-6}$ alkyl;
$R_6$ and $R_7$ are each independently H, D or $C_{1-6}$ alkyl;
$R_8$ and $R_9$ are each independently H, D or $C_{1-6}$ alkyl; or $R_8$ and $R_9$ together with the carbon atom connected thereto form C=O;
$R_{10}$ is $C_{1-6}$ alkyl, $C_{1-6}$ alkoxy, cyano or $SR_{10d}$, wherein the $C_{1-6}$ alkyl and the $C_{1-6}$ alkoxy are optionally substituted with 1 to 3 halogens;
$R_{10d}$ is H, D or $C_{1-6}$ alkyl;
A is

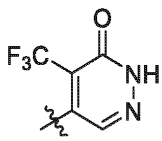

;

B is 5- to 6-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3; and
n is 0, 1, 2 or 3.

One or more embodiments of the present invention provide the use of a compound of general formula (I-2), or a pharmaceutically acceptable salt, a stereoisomer or a deuterated compound thereof as an active ingredient in combination with radiotherapy for the treatment of tumors:

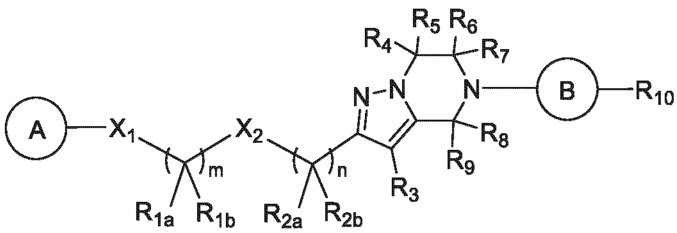

(I-2)

wherein

$X_1$ is NH;
$X_2$ is O;
$R_{1a}$ and $R_{1b}$ are each independently H, D or $C_{1-6}$ alkyl;

$R_{2a}$ and $R_{2b}$ are each independently H, D or $C_{1-6}$ alkyl;
$R_3$ is H, D, $C_{1-6}$ alkyl or halogen, wherein the $C_{1-6}$ alkyl is optionally substituted with 1 to 3 halogens;
$R_4$ and $R_5$ are each independently H, D or $C_{1-6}$ alkyl;
$R_6$ and $R_7$ are each independently H, D or $C_{1-6}$ alkyl;
$R_8$ and $R_9$ are each independently H, D or $C_{1-6}$ alkyl; or $R_8$ and $R_9$ together with the carbon atom connected thereto form C=O;
$R_{10}$ is $C_{1-6}$ alkyl, $C_{1-6}$ alkoxy, cyano or $SR_{10d}$, wherein the $C_{1-6}$ alkyl and the $C_{1-6}$ alkoxy are optionally substituted with 1 to 3 halogens;
$R_{10d}$ is H, D or $C_{1-6}$ alkyl;
A is

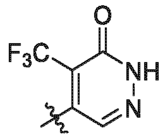
;

B is 5- to 6-membered carbocycle or heterocycle, wherein the heterocycle contains 1 to 3 heteroatoms selected from N, O and S;
m is 1, 2 or 3; and
n is 0, 1, 2 or 3.

One or more embodiments of the present invention provide the use of a compound of general formula (I-2), or a pharmaceutically acceptable salt, a stereoisomer or a deuterated compound thereof as an active ingredient in combination with radiotherapy for the treatment of tumors, wherein

$X_1$ is NH;
$X_2$ is O;
$R_{1a}$ and $R_{1b}$ are each independently H, D or $C_{1-6}$ alkyl;
$R_{2a}$ and $R_{2b}$ are each independently H, D or $C_{1-6}$ alkyl;
$R_3$ is H, D, $C_{1-6}$ alkyl or halogen, wherein the $C_{1-6}$ alkyl is optionally substituted with 1 to 3 halogens;
$R_4$ and $R_5$ are each independently H, D or $C_{1-6}$ alkyl;
$R_6$ and $R_7$ are each independently H, D or $C_{1-6}$ alkyl;
$R_8$ and $R_9$ are each independently H, D or $C_{1-6}$ alkyl; or $R_8$ and $R_9$ together with the carbon atom connected thereto form C=O;
$R_{10}$ is $C_{1-6}$ alkyl, cyano or $SR_{10d}$, wherein the $C_{1-6}$ alkyl is optionally substituted with 1 to 3 halogens;
$R_{10d}$ is H, D or $C_{1-6}$ alkyl;
A is

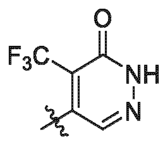
;

B is 5- to 6-membered carbocycle or heterocycle, wherein the heterocycle contains 1 to 3 heteroatoms selected from N, O and S;
m is 1, 2 or 3; and
n is 0, 1, 2 or 3.

One or more embodiments of the present invention provide the use of a compound of general formula (I-2), or a pharmaceutically acceptable salt, a stereoisomer or a deuterated compound thereof as an active ingredient in combination with radiotherapy for the treatment of tumors, wherein

$X_1$ is selected from NH;
$X_2$ is selected from O;
$R_{1a}$ and $R_{1b}$ are each independently selected from H, D or $C_{1-6}$ alkyl;
$R_{2a}$ and $R_{2b}$ are each independently selected from H, D or $C_{1-6}$ alkyl;

$R_3$ is H, D, $C_{1-6}$ alkyl or halogen, wherein the $C_{1-6}$ alkyl is optionally substituted with 1 to 3 halogens;
$R_4$ and $R_5$ are each independently H or D;
$R_6$ and $R_7$ are each independently H or D;
$R_8$ and $R_9$ are each independently H or D;
$R_{10}$ is $CF_3$ or $SR_{10d}$;
$R_{10d}$ is H, D or $C_{1-6}$ alkyl;
A is

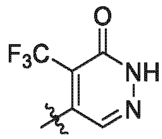
;

B is

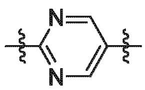
or
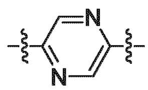
;

m is 1, 2 or 3; and
n is 0, 1, 2 or 3.

One or more embodiments of the present invention provide the use of a compound of general formula (I-2), or a pharmaceutically acceptable salt, a stereoisomer or a deuterated compound thereof as an active ingredient in combination with radiotherapy for the treatment of tumors, wherein

$X_1$ is selected from NH;
$X_2$ is selected from O;
$R_{1a}$ and $R_{1b}$ are each independently selected from H, D or $C_{1-3}$ alkyl;
$R_{2a}$ and $R_{2b}$ are each independently selected from H, D or $C_{1-3}$ alkyl;
$R_3$ is selected from H, D or $CF_3$;
$R_4$ and $R_5$ are each independently selected from H or D;
$R_6$ and $R_7$ are each independently selected from H or D;
$R_8$ and $R_9$ are each independently selected from H or D;
$R_{10}$ is $CF_3$;
A is

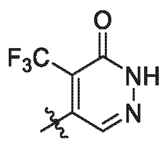
;

B is

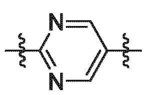
or
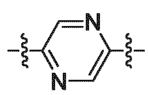
;

m is 1, 2 or 3; and
n is 0, 1 or 2.

One or more embodiments of the present invention provide the use of a compound of general formula (I-3), or a pharmaceutically acceptable salt, a stereoisomer or a deuterated compound thereof as an active ingredient in combination with radiotherapy for the treatment of tumors:

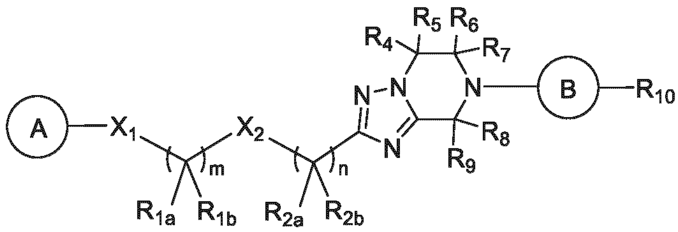

(I-3)

wherein

$X_1$ is NH or 4- to 6-membered heterocycle containing 1 to 3 heteroatoms selected from N and O;
$X_2$ is O;
$R_{1a}$ and $R_{1b}$ are each independently H, D or $C_{1-6}$ alkyl;
$R_{2a}$ and $R_{2b}$ are each independently H, D or $C_{1-6}$ alkyl;
$R_4$ and $R_5$ are each independently H, D or $C_{1-6}$ alkyl;
$R_6$ and $R_7$ are each independently H, D or $C_{1-6}$ alkyl;
$R_8$ and $R_9$ are each independently H, D or $C_{1-6}$ alkyl; or $R_8$ and $R_9$ together with the carbon atom connected thereto form C=O;
$R_{10}$ is $C_{1-6}$ alkyl, $C_{1-6}$ alkoxy, cyano or $SR_{10d}$, wherein the $C_{1-6}$ alkyl and the $C_{1-6}$ alkoxy are optionally substituted with 1 to 3 halogens;
$R_{10d}$ is H, D or $C_{1-6}$ alkyl;
A is

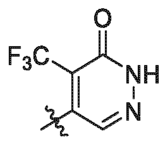

;

B is 5- to 6-membered carbocycle or heterocycle, wherein the heterocycle contains 1 to 3 heteroatoms selected from N, O and S;
m is 1, 2 or 3; and
n is 0, 1, 2 or 3.

One or more embodiments of the present invention provide the use of a compound of general formula (I-3), or a pharmaceutically acceptable salt, a stereoisomer or a deuterated compound thereof as an active ingredient in combination with radiotherapy for the treatment of tumors, wherein

$X_1$ is NH or 4- to 6-membered heterocycle containing 1 to 3 heteroatoms selected from N and O;
$X_2$ is O;
$R_{1a}$ and $R_{1b}$ are each independently H, D or $C_{1-3}$ alkyl;
$R_{2a}$ and $R_{2b}$ are each independently H, D or $C_{1-3}$ alkyl;
$R_4$ and $R_5$ are each independently H, D or $C_{1-3}$ alkyl;
$R_6$ and $R_7$ are each independently H, D or $C_{1-3}$ alkyl;
$R_8$ and $R_9$ are each independently H, D or $C_{1-3}$ alkyl; or $R_8$ and $R_9$ together with the carbon atom connected thereto form C=O;
$R_{10}$ is $C_{1-6}$ alkyl, cyano or $SR_{10d}$, wherein the $C_{1-6}$ alkyl is optionally substituted with 1 to 3 halogens;
$R_{10d}$ is H, D or $C_{1-6}$ alkyl;
A is

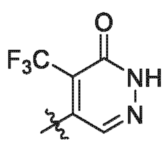

;

B is

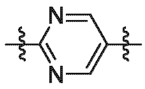 or 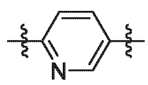 ;

m is 1, 2 or 3; and
n is 0, 1 or 2.

One or more embodiments of the present invention provide the use of a compound of general formula (I-3), or a pharmaceutically acceptable salt, a stereoisomer or a deuterated compound thereof as an active ingredient in combination with radiotherapy for the treatment of tumors, wherein

$X_1$ is NH;
$X_2$ is O;
$R_{1a}$ and $R_{1b}$ are each independently H, D or $C_{1-3}$ alkyl;
$R_{2a}$ and $R_{2b}$ are each independently H or D;
$R_4$ and $R_5$ are each independently H or D;
$R_6$ and $R_7$ are each independently H or D;
$R_8$ and $R_9$ are each independently H or D;
$R_{10}$ is $CF_3$;
A is

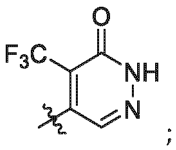 ;

B is

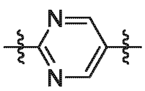 or 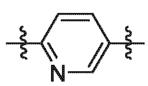 ;

m is 1, 2 or 3; and
n is 0, 1 or 2.

In one or more embodiments of the present invention, the active ingredient is selected from compounds as shown below, or a pharmaceutically acceptable salt, a stereoisomer, or a deuterated compound thereof:

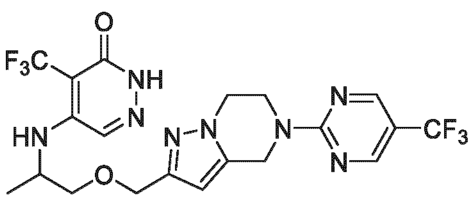 , 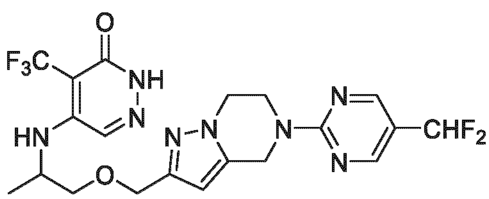 ,

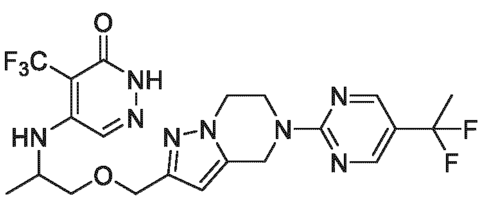 , 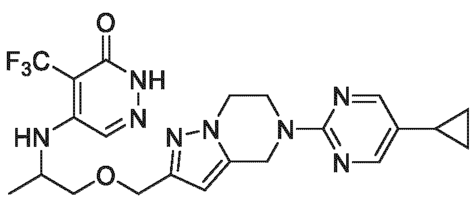 ,

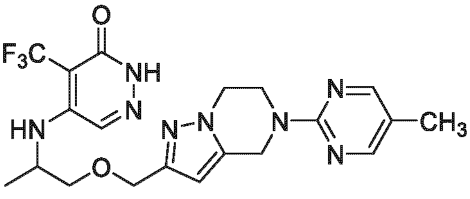
,
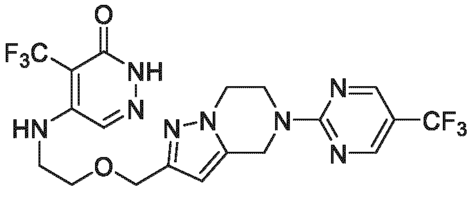
,

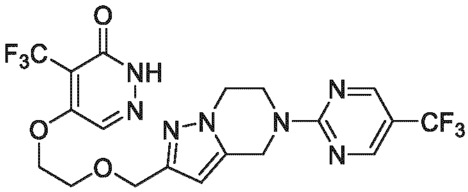
,
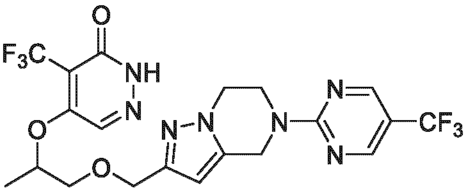
,

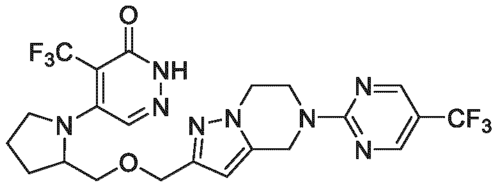
,
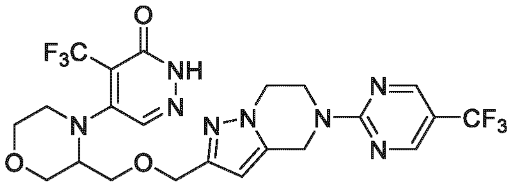
,

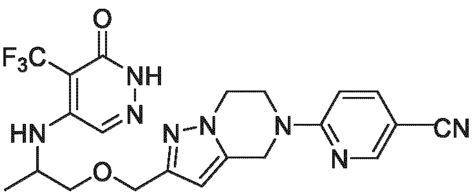
,
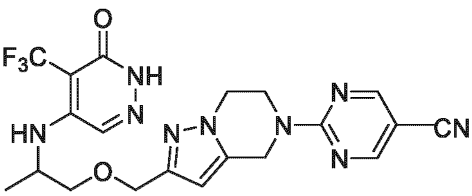
,

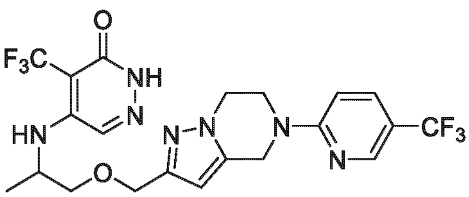
,
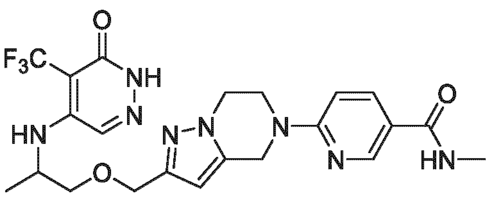
,

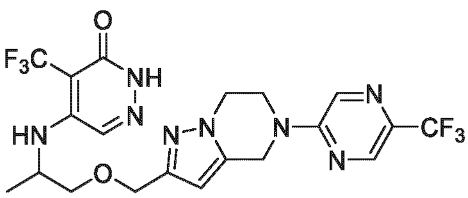
,
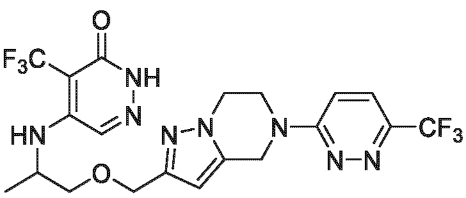
,

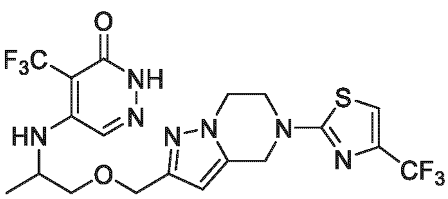
,
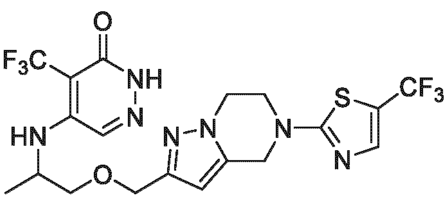
,

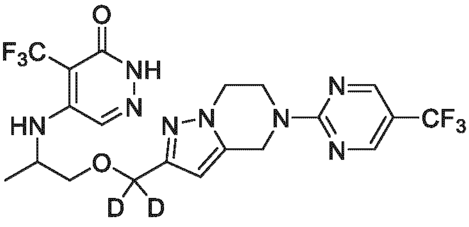,
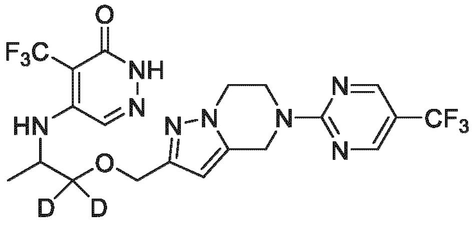,

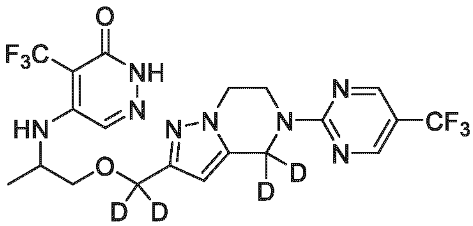,
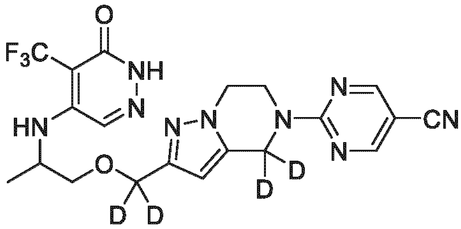,

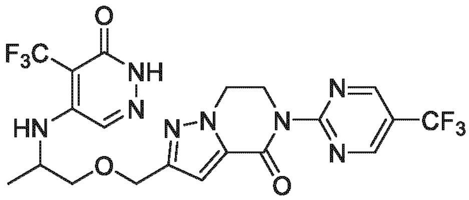,
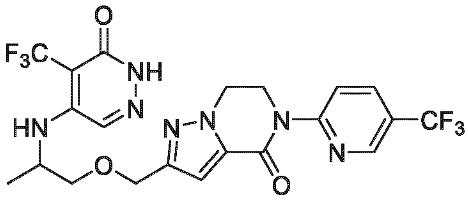,

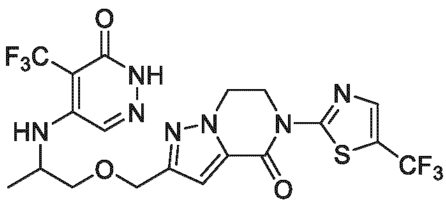,
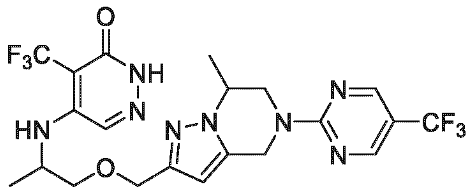,

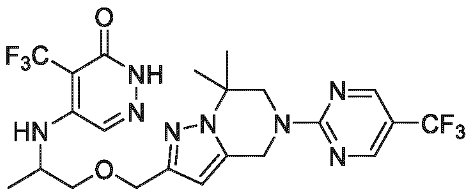,
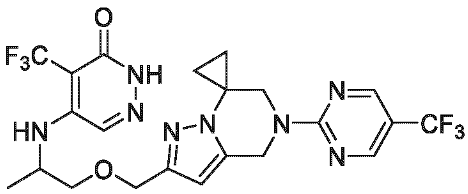,

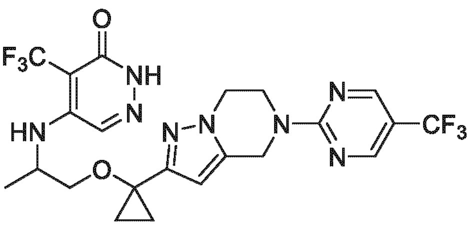,
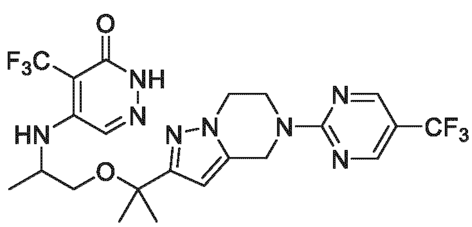,

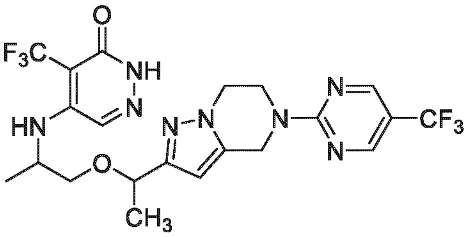

,

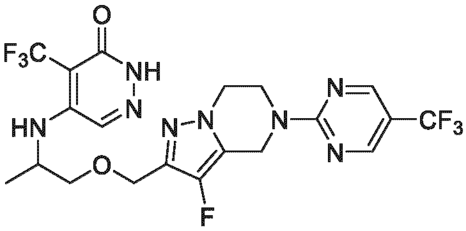

,

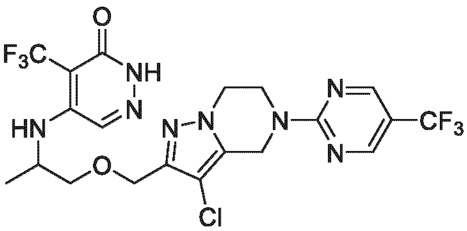

,

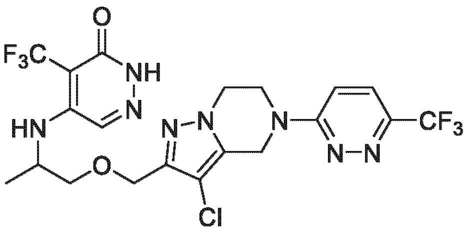

,

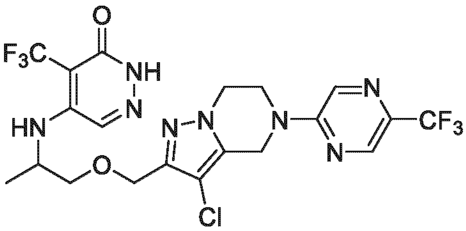

,

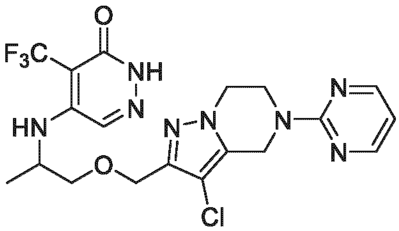

,

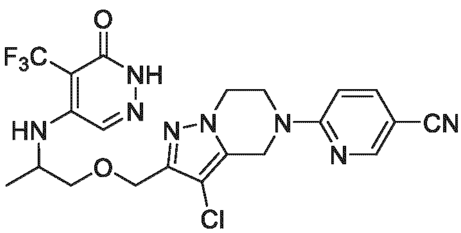

,

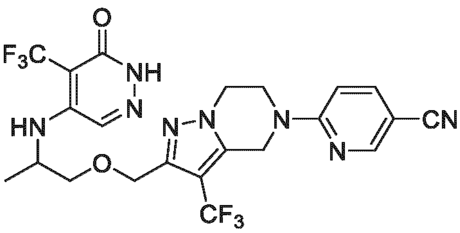

,

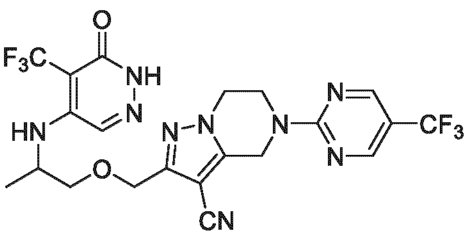

,

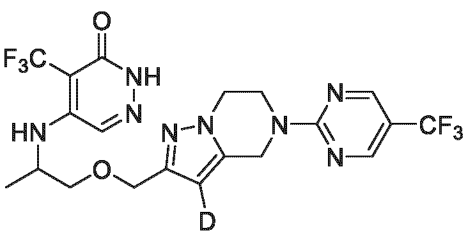

,

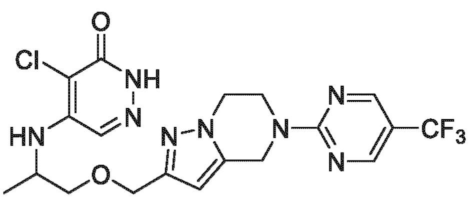

,

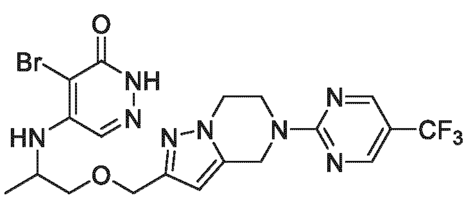

,

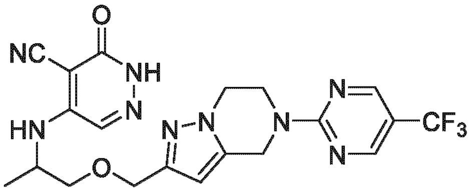
,
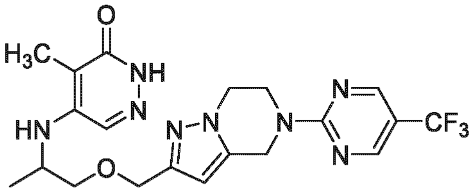
,
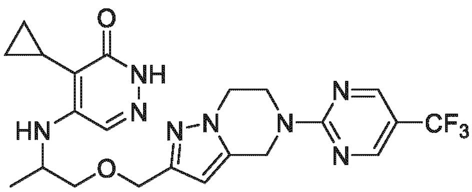
,
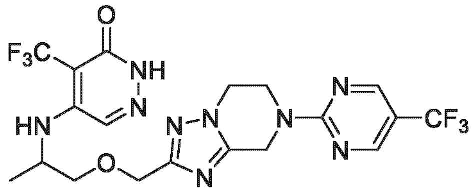
,
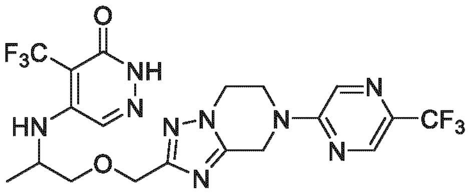
,
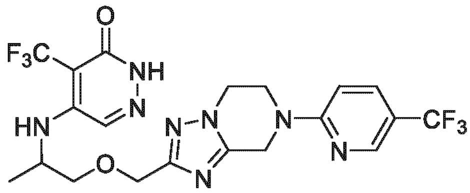
,
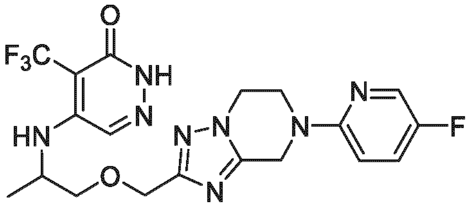
,
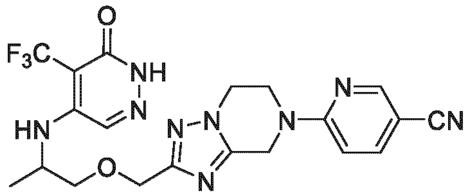
,
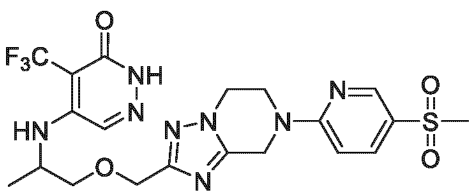
,
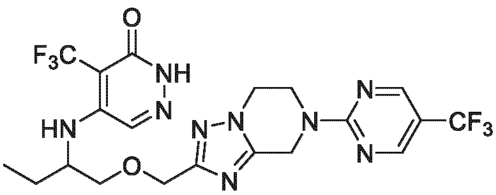
,
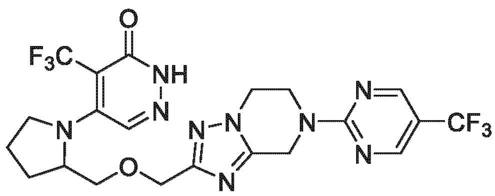
,
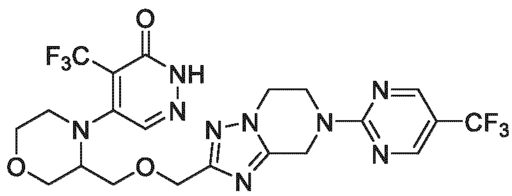
,
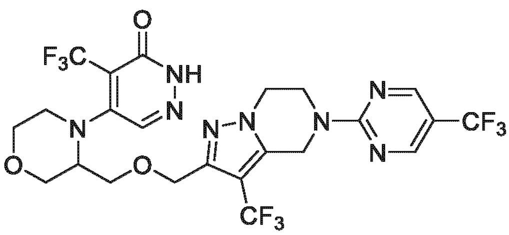
,
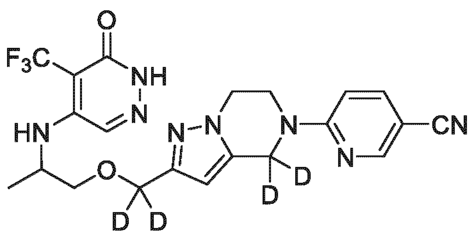
,

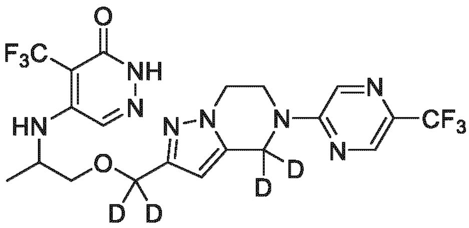
,
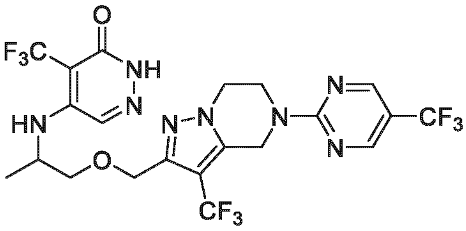
,
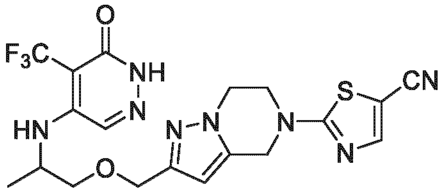
,
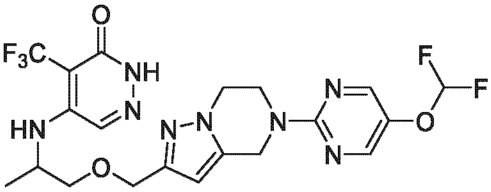
,
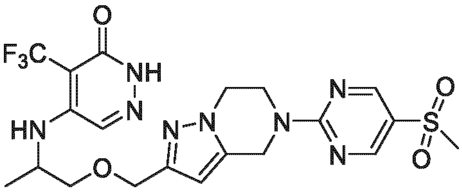
,
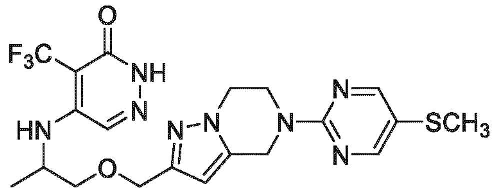
,
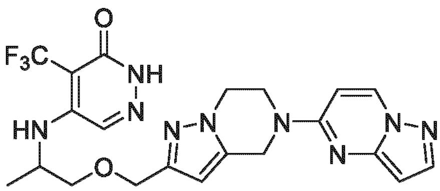
,
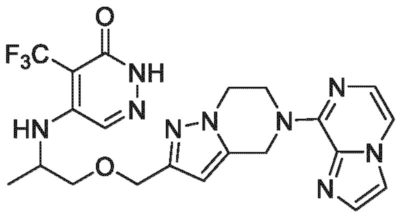
,
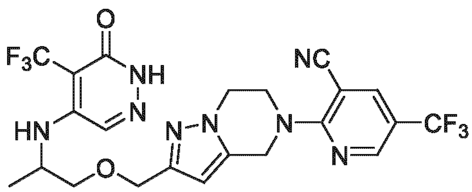
,
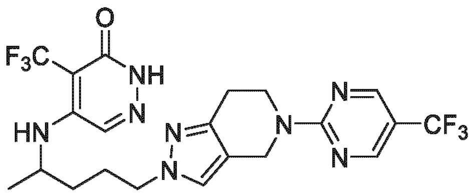
or
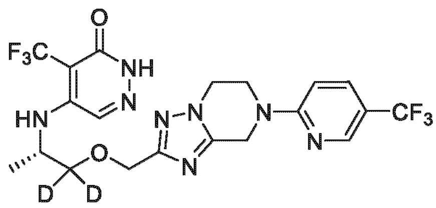
.

In one or more embodiments of the present application, when the active ingredient is applied in combination with radiotherapy, there is a synergistic effect.

In one or more embodiments of the present application, the active ingredient is contained in a pharmaceutical composition further comprising one or more pharmaceutically acceptable excipients, diluents or carriers.

In one or more embodiments of the present application, the total daily dosage of the active ingredient is selected from 50-1500 mg, preferably 100-1000 mg, and more preferably 400-800 mg, as measured by the amount of free base.

In one or more embodiments of the present application, the active ingredient and the radiotherapy are administered simultaneously.

In one or more embodiments of the present application, the active ingredient and the radiotherapy are

administered sequentially, e.g., the active ingredient is administered first, followed by the radiotherapy, alternatively, the radiotherapy is administered first, followed by the active ingredient. The administration intervals may be in hours, days, weeks, or months.

In one or more embodiments of the present application, the active ingredient is given orally at a frequency of thrice daily, twice daily, once daily, once every two days, once every three days, once every four days, once every five days, once every six days, once a week, once every two weeks, once every three weeks, or once every four weeks, preferably twice daily or once daily.

In one or more embodiments of the present application, the tumor is selected from solid tumor.

In one or more embodiments of the present application, the solid tumor is selected from non-small cell lung cancer, head and neck squamous carcinoma, esophageal squamous carcinoma, hormone-receptor-positive (HR+) breast cancer, advanced solid tumor with PARP7 expansion, colon cancer or lung cancer, preferably colon cancer or lung cancer, and more preferably colon cancer.

Advantages of the present invention

The compound ① in combination with radiotherapy has an anti-tumor effect significantly superior to that of radiotherapy alone, and is also well-tolerated and safe. It is shown that when the active ingredient of the present invention is applied in combination with radiotherapy, there is a significant synergistic effect.

Brief Description of the Drawings

FIG. 1 shows the structural formula of the compound ①.
FIG. 2 shows growth curves of tumor volume of tumor-bearing mice.
FIG. 3 shows percent body weight change of tumor-bearing mice.

Detailed Description of Embodiments

The implementation process and beneficial effects of the present invention are described in detail below by way of specific examples, which are intended to help readers better understand the essence and characteristics of the present invention but not to limit the scope of implementation of the present invention.

The present invention will be described in further detail below with reference to the accompanying drawings.

The compound ① in the examples is compound 1 of PCT application PCT/CN2022/094124 and is prepared according to the preparation method therefor.

**Efficacy test in CT26 mouse tumor-bearing model**

**1. Experimental steps**

1.1 Cell culture

Colon cancer cells CT26 were purchased from ATCC for culture under conditions of a DMEM medium supplemented with 10% fetal bovine serum and 1% penicillin and streptomycin, in a cell incubator containing 5% $CO_2$ at 37°C. The cells in an exponential growth phase were digested with trypsin, and the cells were collected and counted, followed by inoculation.

1.2 Subcutaneously-transplanted tumor construction

Female BALB/c mice were selected to constitute a CT26 subcutaneously-transplanted tumor model by subcutaneous injection of tumor cells. When the average tumor volume reached 120-130 $mm^3$, 32 animals were screened for enrollment. The animals were randomly divided into 4 groups based on the tumor volume, namely the blank group, the compound ① group, the irradiated group, and the combined compound ① and irradiation group, with 8 animals per group. The day of the grouping was set as day 0 of the trial (PG-D0). Each irradiated group was given an irradiation dose of 2 Gy for 5 consecutive days starting from D1, and compound ① (125 mpk, BID) administration began on day 1 (D1).

**2. Assay method**

2.1 Tumor volume

The diameter of tumors was measured twice a week using a vernier caliper to calculate the volume of tumors, and a tumor growth curve was plotted. The calculation formula of the tumor volume (V) is:

$$V = 1/2 \times a \times b^2,$$

where a and b represent the long and short diameters of the tumor, respectively.

2.2 Body weight of mice

Mice were weighed at least twice a week during a drug treatment cycle.

**3. Experimental results**

As shown in FIG. 2 and FIG. 3, the anti-tumor effect of compound ① in combination with radiotherapy is significantly superior to that of radiotherapy alone, and is also well-tolerated and safe. It is shown that when the active ingredient of the present invention is applied in combination with radiotherapy, there is a significant synergistic effect.

Specific embodiments have been described in detail in the specification of the present invention. A person skilled in the art should recognize that the embodiments described above are exemplary and cannot be construed as limiting the present invention. Additionally, a person skilled in the art can make several improvements and modifications to the present invention without departing from the principle of the present invention, and the technical solutions obtained based on these improvements and modifications also fall within the scope of protection of the claims of the present invention.

**Claims**

**1.** Use of a compound of formula (I), or a pharmaceutically acceptable salt, a stereoisomer or a deuterated compound thereof as an active ingredient in combination with radiotherapy for the treatment of tumors:

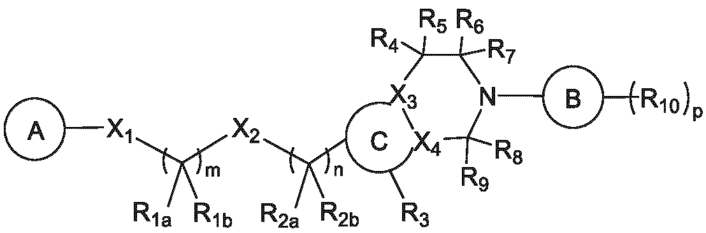

(I)

wherein

$X_1$ is NH, O or 4- to 6-membered heterocycle containing 1 to 3 heteroatoms selected from N and O;
$X_2$ is O or a single bond;
$X_3$ and $X_4$ are each independently C or N;
$R_{1a}$ and $R_{1b}$ are each independently H, D or $C_{1-6}$ alkyl; or $R_{1a}$ and $R_{1b}$ together with the carbon atom connected thereto form 3- to 5-membered cycloalkyl;
$R_{2a}$ and $R_{2b}$ are each independently H, D or $C_{1-6}$ alkyl; or $R_{2a}$ and $R_{2b}$ together with the carbon atom connected thereto form 3- to 5-membered cycloalkyl;
$R_3$ is H, D, $C_{1-6}$ alkyl, halogen or cyano, wherein the $C_{1-6}$ alkyl is optionally substituted with 1 to 3 halogens;
$R_4$ and $R_5$ are each independently H, D or $C_{1-6}$ alkyl; or $R_4$ and $R_5$ together with the carbon atom connected thereto form 3- to 5-membered cycloalkyl;
$R_6$ and $R_7$ are each independently H, D or $C_{1-6}$ alkyl; or $R_6$ and $R_7$ together with the carbon atom connected thereto form C=O;
$R_8$ and $R_9$ are each independently H, D or $C_{1-6}$ alkyl; or $R_8$ and $R_9$ together with the carbon atom connected

thereto form C=O; or $R_8$ and $R_9$ together with the carbon atom connected thereto form 3- to 5-membered cycloalkyl;

each $R_{10}$ is independently $C_{1-6}$ alkyl, $C_{1-6}$ alkoxy, $CONR_{10a}R_{10b}$, halogen, cyano, $S(O)_2R_{10c}$, $SR_{10d}$ or 3-to 5-membered cycloalkyl, wherein the $C_{1-6}$ alkyl and $C_{1-6}$ alkoxy are optionally substituted with 1 to 3 halogens;

$R_{10a}$, $R_{10b}$, $R_{10c}$ and $R_{10d}$ are each independently H, D or $C_{1-6}$ alkyl;

A is

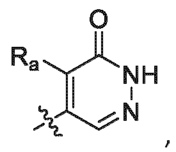

and $R_a$ is $C_{1-6}$ alkyl, $C_{3-5}$ cycloalkyl, halogen or cyano, wherein the $C_{1-6}$ alkyl is optionally substituted with 1 to 3 halogens;

B is 5- to 10-membered carbocycle or heterocycle, wherein the heterocycle contains 1 to 3 heteroatoms selected from N, O and S;

C is 5- to 6-membered heterocycle containing 1 to 3 heteroatoms of N;

m is 1, 2 or 3;

n is 0, 1, 2 or 3; and

p is 0, 1, 2 or 3.

**2.** The use according to claim 1, wherein the active ingredient is a compound of formula (I), or a pharmaceutically acceptable salt, a stereoisomer, or a deuterated compound thereof, wherein $X_1$ is NH, O or 4- to 6-membered heterocycle containing 1 to 3 heteroatoms selected from N and O;

$X_2$ is O or a single bond;

$X_3$ and $X_4$ are each independently C or N;

$R_{1a}$ and $R_{1b}$ are each independently H, D or $C_{1-6}$ alkyl;

$R_{2a}$ and $R_{2b}$ are each independently H, D or $C_{1-6}$ alkyl; or $R_{2a}$ and $R_{2b}$ together with the carbon atom connected thereto form 3- to 5-membered cycloalkyl;

$R_3$ is H, D, $C_{1-6}$ alkyl, halogen or cyano, wherein the $C_{1-6}$ alkyl is optionally substituted with 1 to 3 halogens;

$R_4$ and $R_5$ are each independently H, D or $C_{1-6}$ alkyl; or $R_4$ and $R_5$ together with the carbon atom connected thereto form 3- to 5-membered cycloalkyl;

$R_6$ and $R_7$ are each independently H, D or $C_{1-6}$ alkyl;

$R_8$ and $R_9$ are each independently H, D or $C_{1-6}$ alkyl; or $R_8$ and $R_9$ together with the carbon atom connected thereto form C=O;

each $R_{10}$ is independently $C_{1-6}$ alkyl, $C_{1-6}$ alkoxy, $CONR_{10a}R_{10b}$, halogen, cyano, $S(O)_2R_{10c}$, $SR_{10d}$ or 3-to 5-membered cycloalkyl, wherein the $C_{1-6}$ alkyl and $C_{1-6}$ alkoxy are optionally substituted with 1 to 3 halogens;

$R_{10a}$, $R_{10b}$, $R_{10c}$ and $R_{10d}$ are each independently H, D or $C_{1-6}$ alkyl;

A is

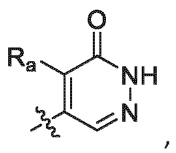

and $R_a$ is $C_{1-6}$ alkyl, $C_{3-5}$ cycloalkyl, halogen or cyano, wherein the $C_{1-6}$ alkyl is optionally substituted with 1 to 3 halogens;

B is 5- to 6-membered carbocycle or heterocycle, wherein the heterocycle contains 1 to 3 heteroatoms selected from N, O and S;

C is 5- to 6-membered heterocycle containing 1 to 3 heteroatoms of N;

m is 1, 2 or 3;

n is 0, 1, 2 or 3; and

p is 0, 1, 2 or 3.

**3.** The use according to claim 2, wherein the active ingredient is a compound of formula (I-1), or a pharmaceutically

acceptable salt, a stereoisomer, or a deuterated compound thereof, wherein

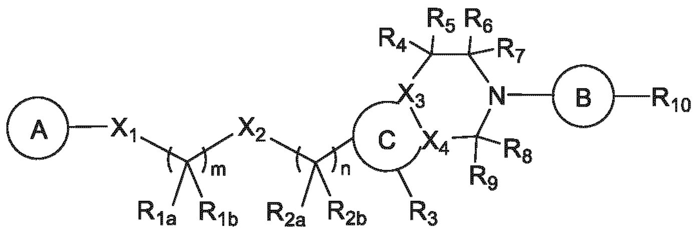

(I-1)

wherein

$X_1$ is NH or 4- to 6-membered heterocycle containing 1 to 3 heteroatoms selected from N and O;
$X_2$ is O;
$X_3$ and $X_4$ are each independently C or N;
$R_{1a}$ and $R_{1b}$ are each independently H, D or $C_{1-6}$ alkyl;
$R_{2a}$ and $R_{2b}$ are each independently H, D or $C_{1-6}$ alkyl;
$R_3$ is H, D, $C_{1-6}$ alkyl or halogen, wherein the $C_{1-6}$ alkyl is optionally substituted with 1 to 3 halogens;
$R_4$ and $R_5$ are each independently H, D or $C_{1-6}$ alkyl;
$R_6$ and $R_7$ are each independently H, D or $C_{1-6}$ alkyl;
$R_8$ and $R_9$ are each independently H, D or $C_{1-6}$ alkyl; or $R_8$ and $R_9$ together with the carbon atom connected thereto form C=O;
$R_{10}$ is $C_{1-6}$ alkyl, $C_{1-6}$ alkoxy, cyano or $SR_{10d}$, wherein the $C_{1-6}$ alkyl and the $C_{1-6}$ alkoxy are optionally substituted with 1 to 3 halogens;
$R_{10d}$ is H, D or $C_{1-6}$ alkyl;
A is

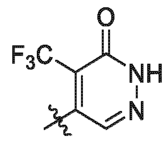

;

B is 5- to 6-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3; and
n is 0, 1, 2 or 3.

**4.** The use according to claim 3, wherein the active ingredient is a compound of formula (I-2), or a pharmaceutically acceptable salt, a stereoisomer, or a deuterated compound thereof, wherein

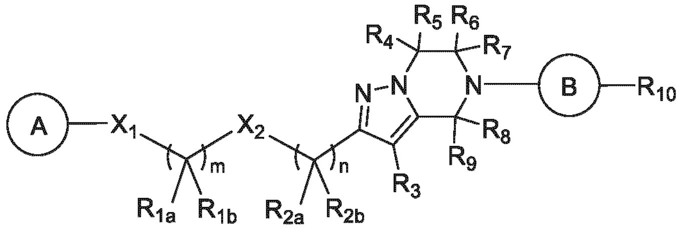

(I-2)

wherein

$X_1$ is NH;

$X_2$ is O;
$R_{1a}$ and $R_{1b}$ are each independently H, D or $C_{1-6}$ alkyl;
$R_{2a}$ and $R_{2b}$ are each independently H, D or $C_{1-6}$ alkyl;
$R_3$ is H, D, $C_{1-6}$ alkyl or halogen, wherein the $C_{1-6}$ alkyl is optionally substituted with 1 to 3 halogens;
$R_4$ and $R_5$ are each independently H, D or $C_{1-6}$ alkyl;
$R_6$ and $R_7$ are each independently H, D or $C_{1-6}$ alkyl;
$R_8$ and $R_9$ are each independently H, D or $C_{1-6}$ alkyl; or $R_8$ and $R_9$ together with the carbon atom connected thereto form C=O;
$R_{10}$ is $C_{1-6}$ alkyl, $C_{1-6}$ alkoxy, cyano or $SR_{10d}$, wherein the $C_{1-6}$ alkyl and the $C_{1-6}$ alkoxy are optionally substituted with 1 to 3 halogens;
$R_{10d}$ is H, D or $C_{1-6}$ alkyl;
A is

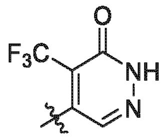
;

B is 5- to 6-membered carbocycle or heterocycle, wherein the heterocycle contains 1 to 3 heteroatoms selected from N, O and S;
m is 1, 2 or 3; and
n is 0, 1, 2 or 3.

**5.** The use according to claim 4, wherein the active ingredient is a compound of formula (I-2), or a pharmaceutically acceptable salt, a stereoisomer, or a deuterated compound thereof, wherein

$X_1$ is NH;
$X_2$ is O;
$R_{1a}$ and $R_{1b}$ are each independently H, D or $C_{1-6}$ alkyl;
$R_{2a}$ and $R_{2b}$ are each independently H, D or $C_{1-6}$ alkyl;
$R_3$ is H, D, $C_{1-6}$ alkyl or halogen, wherein the $C_{1-6}$ alkyl is optionally substituted with 1 to 3 halogens;
$R_4$ and $R_5$ are each independently H, D or $C_{1-6}$ alkyl;
$R_6$ and $R_7$ are each independently H, D or $C_{1-6}$ alkyl;
$R_8$ and $R_9$ are each independently H, D or $C_{1-6}$ alkyl; or $R_8$ and $R_9$ together with the carbon atom connected thereto form C=O;
$R_{10}$ is $C_{1-6}$ alkyl, cyano or $SR_{10d}$, wherein the $C_{1-6}$ alkyl is optionally substituted with 1 to 3 halogens;
$R_{10d}$ is H, D or $C_{1-6}$ alkyl;
A is

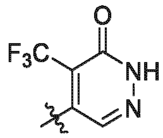
;

B is 5- to 6-membered carbocycle or heterocycle, wherein the heterocycle contains 1 to 3 heteroatoms selected from N, O and S;
m is 1, 2 or 3; and
n is 0, 1, 2 or 3.

**6.** The use according to claim 5, wherein the active ingredient is a compound of formula (I-2), or a pharmaceutically acceptable salt, a stereoisomer, or a deuterated compound thereof, wherein

$X_1$ is selected from NH;
$X_2$ is selected from O;
$R_{1a}$ and $R_{1b}$ are each independently selected from H, D or $C_{1-6}$ alkyl;
$R_{2a}$ and $R_{2b}$ are each independently selected from H, D or $C_{1-6}$ alkyl;

$R_3$ is H, D, $C_{1-6}$ alkyl or halogen, wherein the $C_{1-6}$ alkyl is optionally substituted with 1 to 3 halogens;
$R_4$ and $R_5$ are each independently H or D;
$R_6$ and $R_7$ are each independently H or D;
$R_8$ and $R_9$ are each independently H or D;
$R_{10}$ is $CF_3$ or $SR_{10d}$;
$R_{10d}$ is H, D or $C_{1-6}$ alkyl;
A is

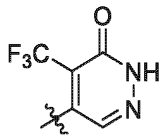
;

B is

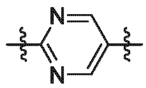
or
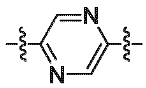
;

m is 1, 2 or 3; and
n is 0, 1, 2 or 3.

**7.** The use according to claim 6, wherein the active ingredient is a compound of formula (I-2), or a pharmaceutically acceptable salt, a stereoisomer, or a deuterated compound thereof, wherein

$X_1$ is selected from NH;
$X_2$ is selected from O;
$R_{1a}$ and $R_{1b}$ are each independently selected from H, D or $C_{1-3}$ alkyl;
$R_{2a}$ and $R_{2b}$ are each independently selected from H, D or $C_{1-3}$ alkyl;
$R_3$ is selected from H, D or $CF_3$;
$R_4$ and $R_5$ are each independently selected from H or D;
$R_6$ and $R_7$ are each independently selected from H or D;
$R_8$ and $R_9$ are each independently selected from H or D;
$R_{10}$ is $CF_3$;
A is

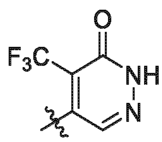
;

B is

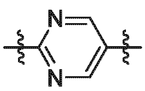
or
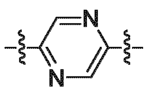
;

m is 1, 2 or 3; and
n is 0, 1 or 2.

**8.** The use according to claim 1, wherein the active ingredient is a compound of formula (I-3), or a pharmaceutically acceptable salt, a stereoisomer, or a deuterated compound thereof, wherein

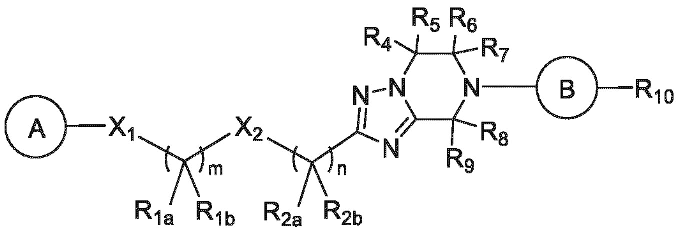

(I-3)

wherein

$X_1$ is NH or 4- to 6-membered heterocycle containing 1 to 3 heteroatoms selected from N and O;
$X_2$ is O;
$R_{1a}$ and $R_{1b}$ are each independently H, D or $C_{1-6}$ alkyl;
$R_{2a}$ and $R_{2b}$ are each independently H, D or $C_{1-6}$ alkyl;
$R_4$ and $R_5$ are each independently H, D or $C_{1-6}$ alkyl;
$R_6$ and $R_7$ are each independently H, D or $C_{1-6}$ alkyl;
$R_8$ and $R_9$ are each independently H, D or $C_{1-6}$ alkyl; or $R_8$ and $R_9$ together with the carbon atom connected thereto form C=O;
$R_{10}$ is $C_{1-6}$ alkyl, $C_{1-6}$ alkoxy, cyano or $SR_{10d}$, wherein the $C_{1-6}$ alkyl and the $C_{1-6}$ alkoxy are optionally substituted with 1 to 3 halogens;
$R_{10d}$ is H, D or $C_{1-6}$ alkyl;
A is

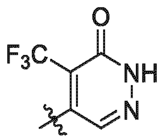
;

B is 5- to 6-membered carbocycle or heterocycle, wherein the heterocycle contains 1 to 3 heteroatoms selected from N, O and S;
m is 1, 2 or 3; and
n is 0, 1, 2 or 3.

**9.** The use according to claim 8, wherein the active ingredient is a compound of formula (I-3), or a pharmaceutically acceptable salt, a stereoisomer, or a deuterated compound thereof, wherein

$X_1$ is NH or 4- to 6-membered heterocycle containing 1 to 3 heteroatoms selected from N and O;
$X_2$ is O;
$R_{1a}$ and $R_{1b}$ are each independently H, D or $C_{1-3}$ alkyl;
$R_{2a}$ and $R_{2b}$ are each independently H, D or $C_{1-3}$ alkyl;
$R_4$ and $R_5$ are each independently H, D or $C_{1-3}$ alkyl;
$R_6$ and $R_7$ are each independently H, D or $C_{1-3}$ alkyl;
$R_8$ and $R_9$ are each independently H, D or $C_{1-3}$ alkyl; or $R_8$ and $R_9$ together with the carbon atom connected thereto form C=O;
$R_{10}$ is $C_{1-6}$ alkyl, cyano or $SR_{10d}$, wherein the $C_{1-6}$ alkyl is optionally substituted with 1 to 3 halogens;
$R_{10d}$ is H, D or $C_{1-6}$ alkyl;
A is

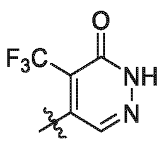
;

B is

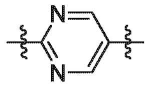 or 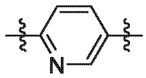 ;

m is 1, 2 or 3; and
n is 0, 1 or 2.

**10.** The use according to claim 9, wherein the active ingredient is a compound of formula (I-3), or a pharmaceutically acceptable salt, a stereoisomer, or a deuterated compound thereof, wherein

$X_1$ is NH;
$X_2$ is O;
$R_{1a}$ and $R_{1b}$ are each independently H, D or $C_{1-3}$ alkyl;
$R_{2a}$ and $R_{2b}$ are each independently H or D;
$R_4$ and $R_5$ are each independently H or D;
$R_6$ and $R_7$ are each independently H or D;
$R_8$ and $R_9$ are each independently H or D;
$R_{10}$ is $CF_3$;
A is

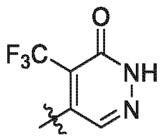 ;

B is

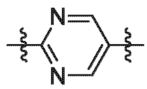 or 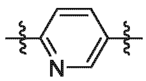 ;

m is 1, 2 or 3; and
n is 0, 1 or 2.

**11.** The use according to any one of claims 1 to 10, wherein the active ingredient is selected from compounds as shown below, or a pharmaceutically acceptable salt, a stereoisomer, or a deuterated compound thereof:

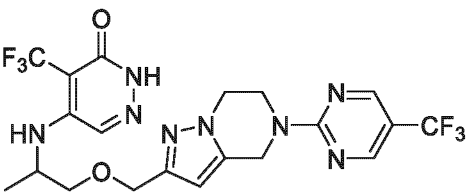 , 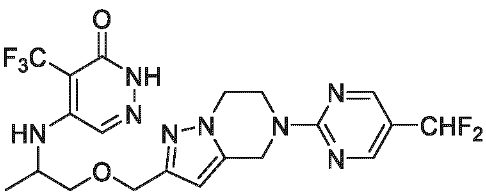 ,

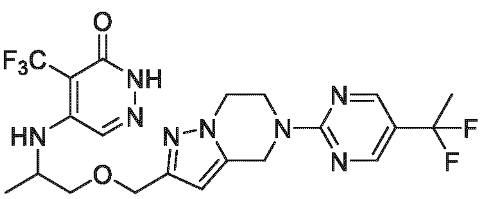 , 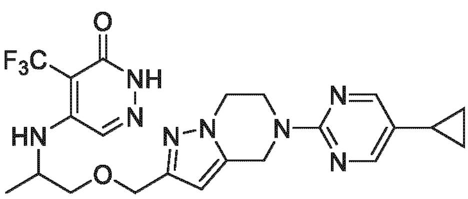 ,

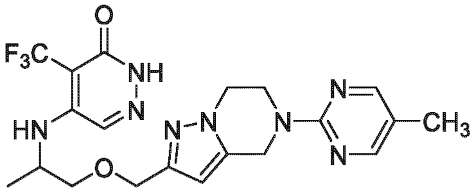,
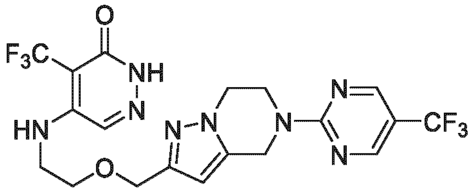,

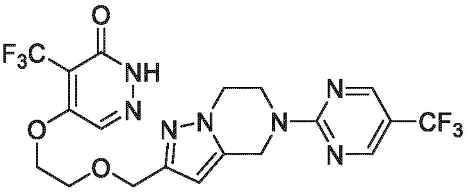,
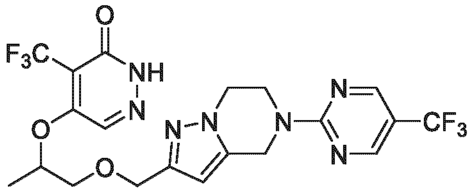,

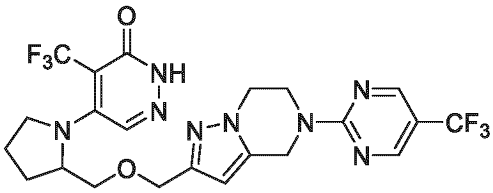,
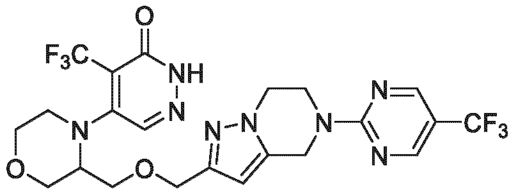,

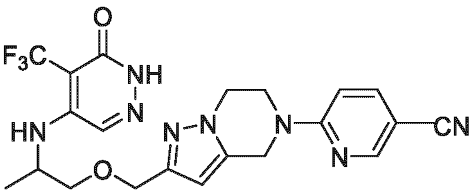,
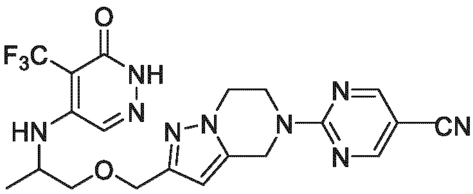,

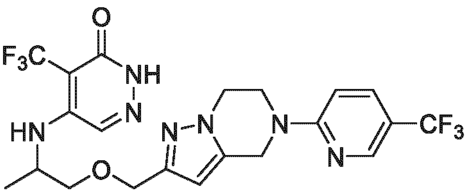,
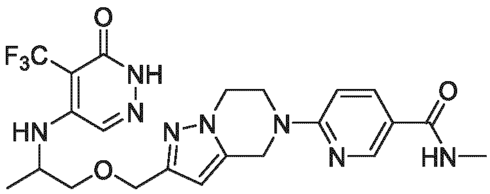,

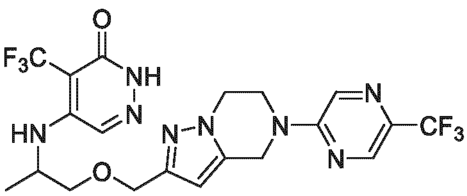,
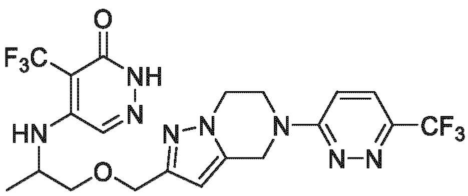,

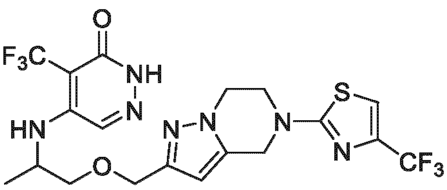,
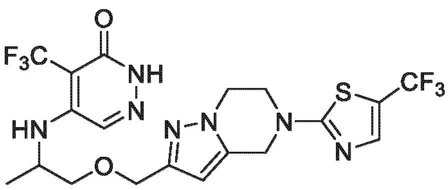,

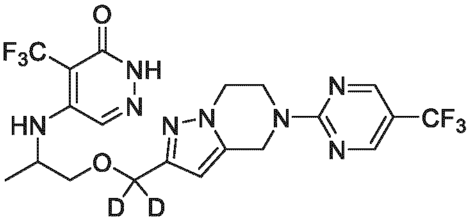,
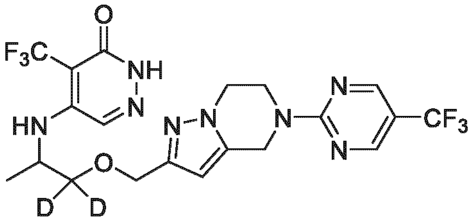,
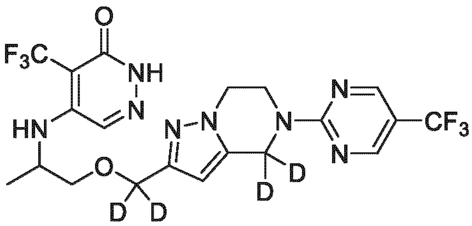,
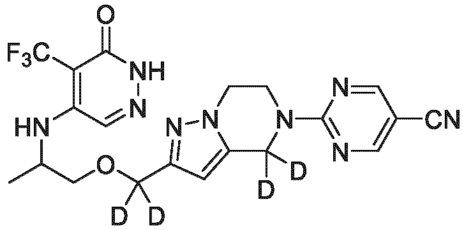,
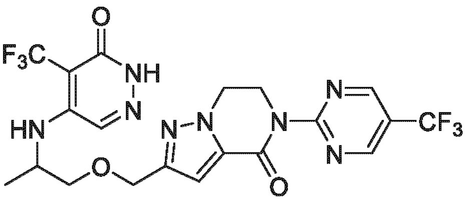,
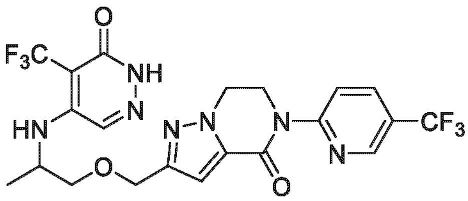,
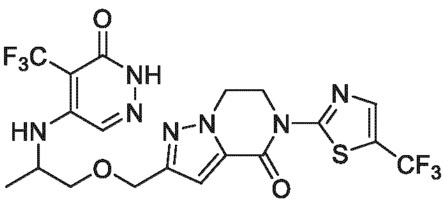,
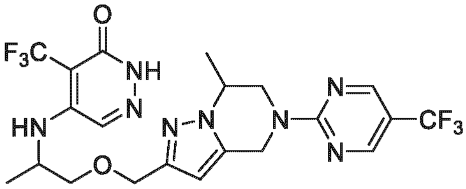,
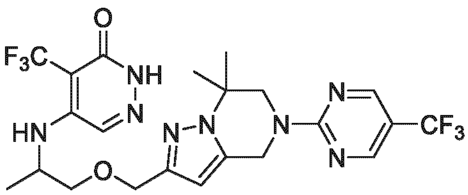,
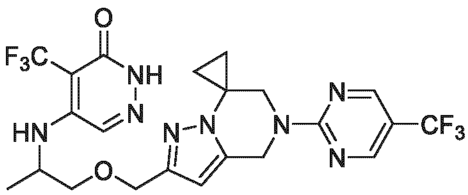,
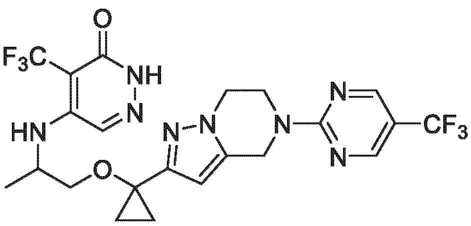,
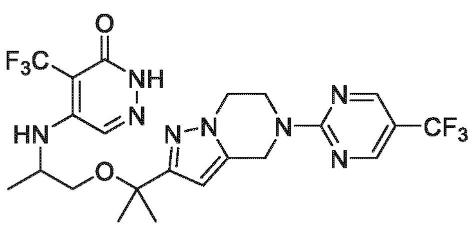,

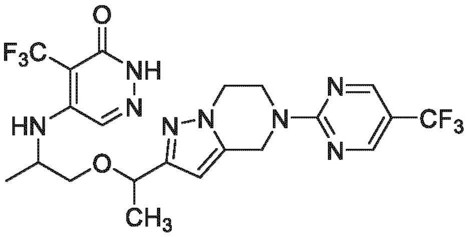
,
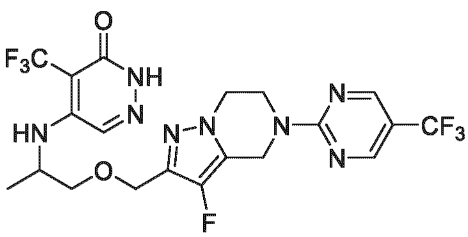
,

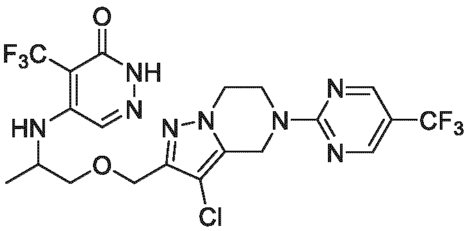
,
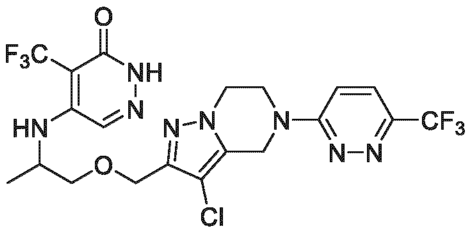
,

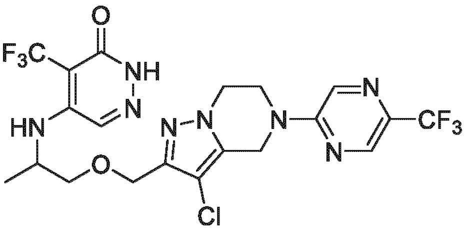
,
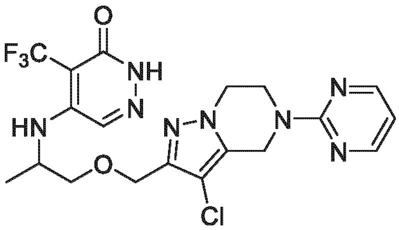
,

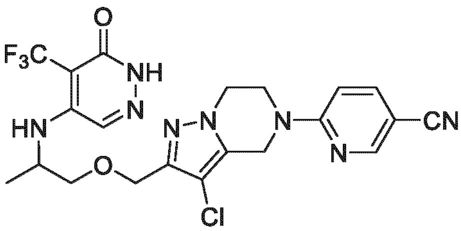
,
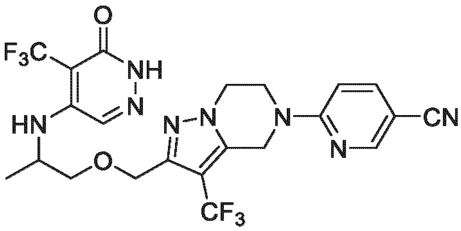
,

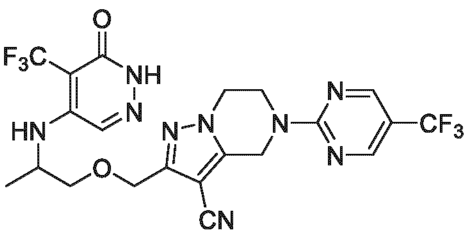
,
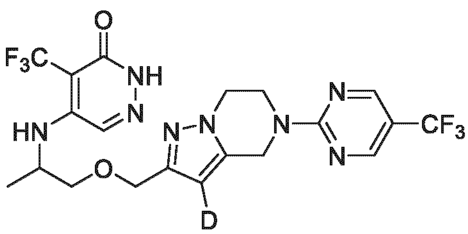
,

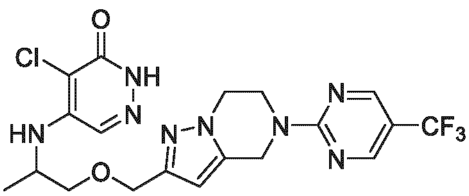
,
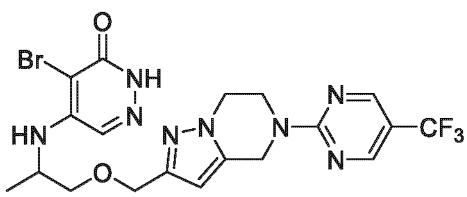
,

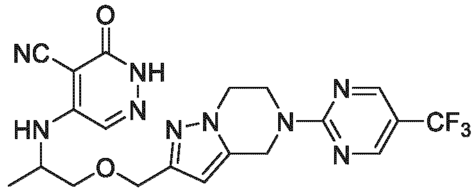 , 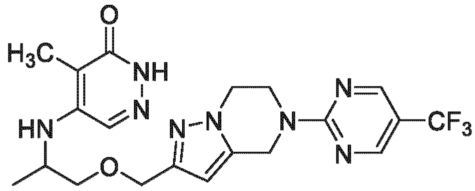 ,

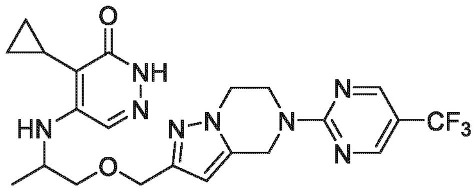 , 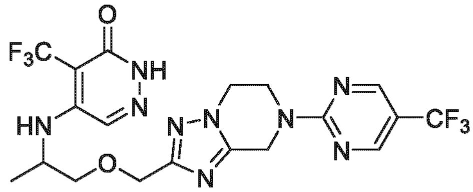 ,

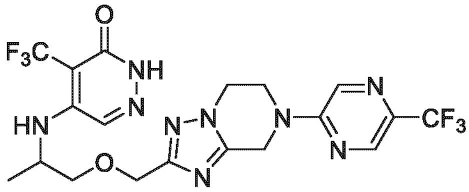 , 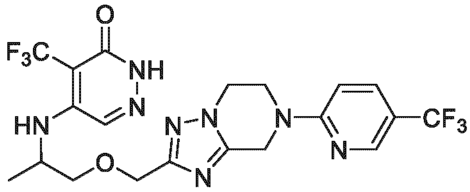 ,

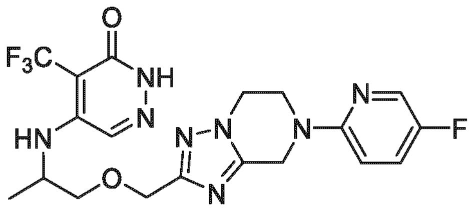 , 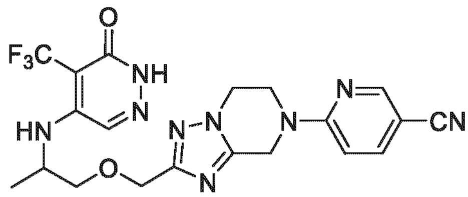 ,

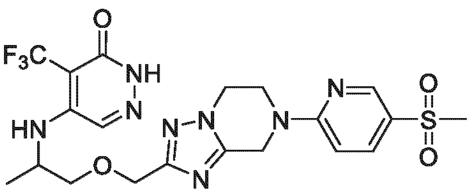 , 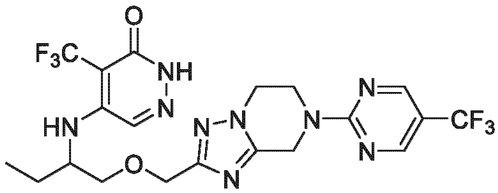 ,

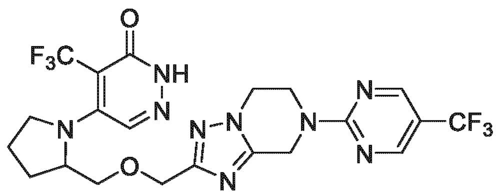 , 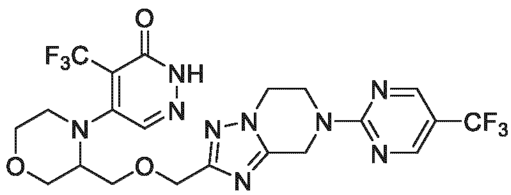 ,

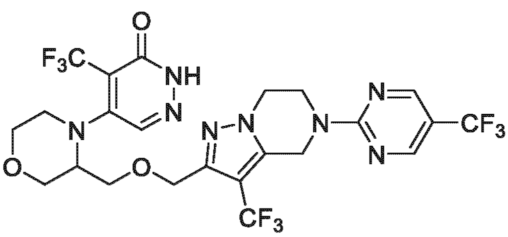 , 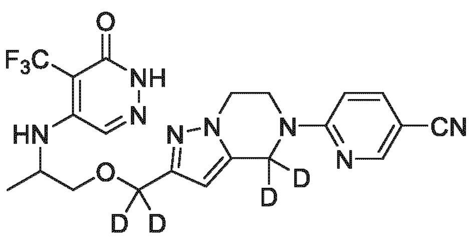 ,

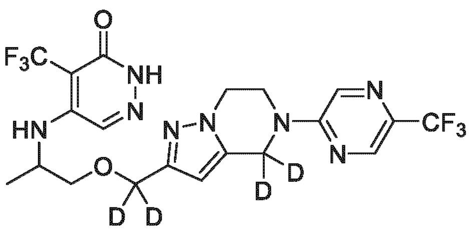,
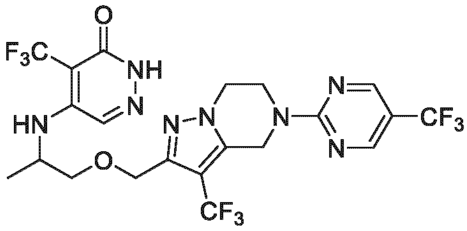,

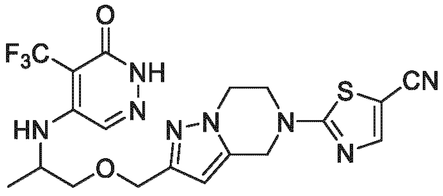,
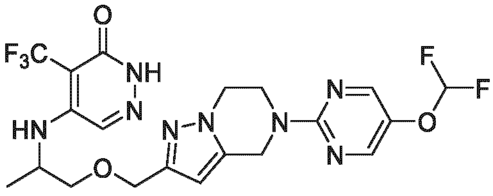,

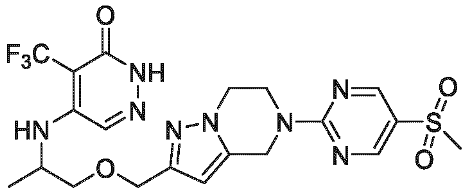,
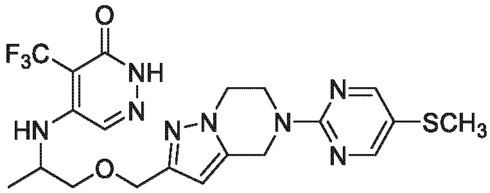,

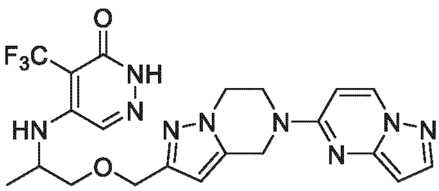,
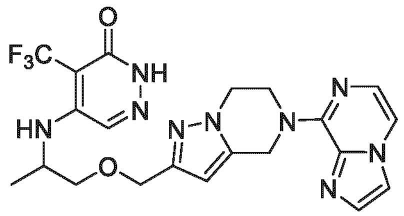,

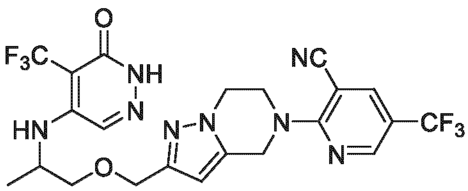,
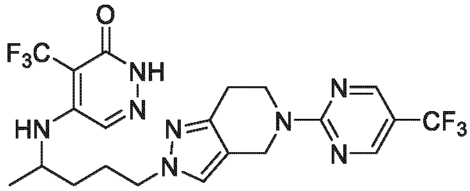

or

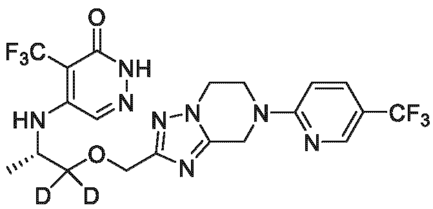.

12. The use according to any one of claims 1 to 11, wherein the active ingredient is contained in a pharmaceutical composition further comprising one or more pharmaceutically acceptable excipients, diluents or carriers.

13. The use according to any one of claims 1 to 12, wherein the total daily dosage of the active ingredient is selected from 50-1500 mg, preferably 100-1000 mg, and more preferably 400-800 mg, as measured by the amount of free base.

14. The use according to any one of claims 1 to 13, wherein the active ingredient and the radiotherapy are administered

simultaneously.

**15.** The use according to any one of claims 1 to 13, wherein the active ingredient and the radiotherapy are administered sequentially.

**16.** The use according to any one of claims 1 to 15, wherein the active ingredient is given orally at a frequency of twice daily or once daily.

**17.** The use according to any one of claims 1 to 16, wherein the tumor is selected from solid tumor.

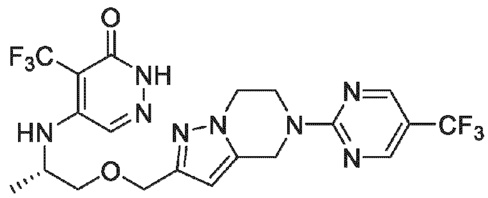

Figure 1

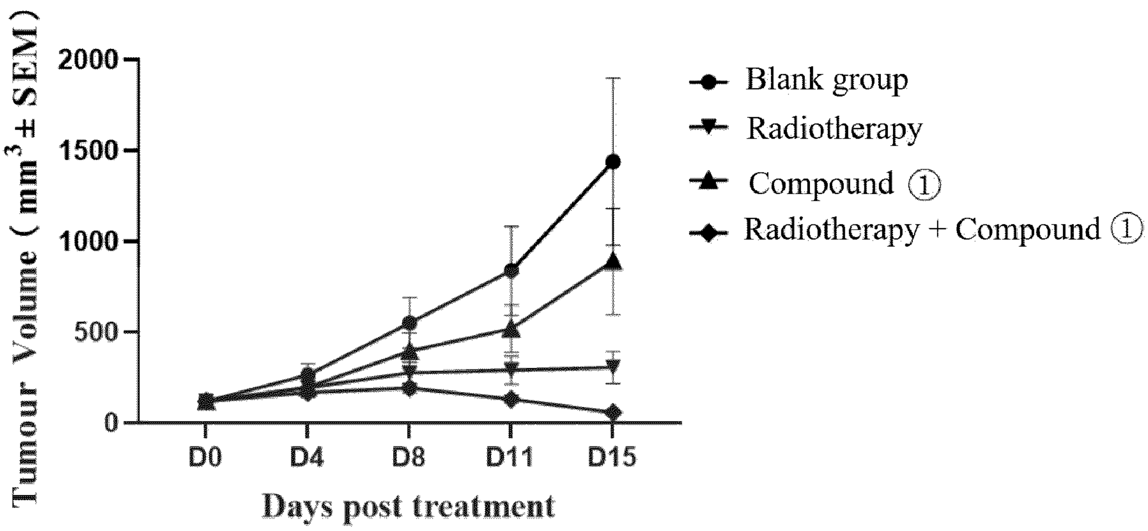


Growth curves of tumor volume of tumor-bearing mice.

Figure 2

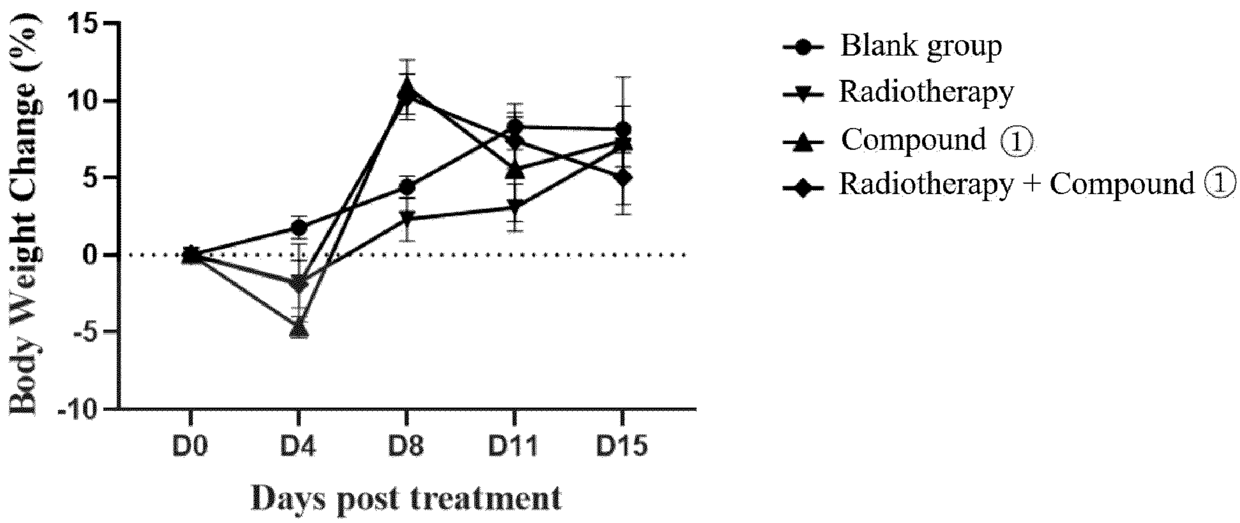


Percent body weight change of tumor-bearing mice.

Figure 3

**INTERNATIONAL SEARCH REPORT**

International application No.
**PCT/CN2023/110230**

**A. CLASSIFICATION OF SUBJECT MATTER**

A61K31/4985(2006.01)i； C07D471/04(2006.01)i； A61P35/00(2006.01)i

According to International Patent Classification (IPC) or to both national classification and IPC

**B. FIELDS SEARCHED**

Minimum documentation searched (classification system followed by classification symbols)

IPC:A61K,C07D,A61P

Documentation searched other than minimum documentation to the extent that such documents are included in the fields searched

Electronic data base consulted during the international search (name of data base and, where practicable, search terms used)

CNTXT, WPABS, WPABSC, ENTXTC, OETXT, VEN, NPABS, 中国药物专利数据库, CHINESE PHARMACEUTICAL PATENT DATABASE, CNKI, 百度学术, Baidu Scholar, 超星读秀学术(CN), CHAOXING DUXIU SCHOLAR, 万方(CN), WANFANG, Medline, ISI-WEB OF SCIENCE, REGISTRY, CAPLUS: 结构式检索, structural formula search, 癌, 肿瘤, 四氢吡唑, 吡嗪, 哒嗪, 嘧啶, pyrimidin+, tetrahydropyrazolo+, pyrazin+, pyridazin+, cancer, tumor, tumour, neoplasms, leukemia

**C. DOCUMENTS CONSIDERED TO BE RELEVANT**

| Category* | Citation of document, with indication, where appropriate, of the relevant passages | Relevant to claim No. |
|---|---|---|
| PX | WO 2022242750 A1 (CHENGDU BAIYU PHARMACEUTICAL CO., LTD.) 24 November 2022 (2022-11-24)<br>see description, pages 10-14 and 21, and claims 1-18 | 1-17 |
| A | CN 112424188 A (RIBON THERAPEUTICS INC.) 26 February 2021 (2021-02-26)<br>see description, paragraphs 0564-0570 and 0630-3781, and claims 1-443 | 1-17 |
| A | CN 112812113 A (HANGZHOU HERTZ PHARMACEUTICAL CO., LTD.) 18 May 2021 (2021-05-18)<br>see claims 1-17 | 1-17 |
| A | CN 113906031 A (JANSSEN SCIENCES IRELAND UC) 07 January 2022 (2022-01-07)<br>see claims 1-33 | 1-17 |
| A | US 2011230472 A1 (SHIONOGI & CO., LTD.) 22 September 2011 (2011-09-22)<br>see abstract, and claims 1-20 | 1-17 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 November 2023** | **13 November 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/110230**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-17**
because they relate to subject matter not required to be searched by this Authority, namely:

These claims relate to a method for treating diseases, which falls within subject matter for which no search is required (PCT Rule 39.1(iv)); however, a search is still conducted for a corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/110230**

| Patent document cited in search report | | | Publication date (day/month/year) | Patent family member(s) | | | Publication date (day/month/year) |
|---|---|---|---|---|---|---|---|
| WO | 2022242750 | A1 | 24 November 2022 | TW | 202246277 | A | 01 December 2022 |
| CN | 112424188 | A | 26 February 2021 | ECSP | 20069404 | A | 29 January 2021 |
| | | | | RS | 64283 | B1 | 31 July 2023 |
| | | | | PT | 3788040 | T | 12 July 2023 |
| | | | | US | 11566020 | B1 | 31 January 2023 |
| | | | | US | 2019330194 | A1 | 31 October 2019 |
| | | | | US | 10550105 | B2 | 04 February 2020 |
| | | | | AR | 121419 | A1 | 08 June 2022 |
| | | | | SI | 3788040 | T1 | 31 July 2023 |
| | | | | FI | 3788040 | T3 | 13 June 2023 |
| | | | | IL | 278116 | A | 30 November 2020 |
| | | | | MA | 52486 | A | 10 March 2021 |
| | | | | MA | 52486 | B1 | 28 April 2023 |
| | | | | EP | 3788040 | A1 | 10 March 2021 |
| | | | | EP | 3788040 | B1 | 12 April 2023 |
| | | | | US | 2023192664 | A1 | 22 June 2023 |
| | | | | KR | 20210014108 | A | 08 February 2021 |
| | | | | JP | 2021523104 | A | 02 September 2021 |
| | | | | JP | 6942896 | B2 | 29 September 2021 |
| | | | | SG | 11202010183 | XA | 27 November 2020 |
| | | | | BR | 112020022006 | A2 | 26 January 2021 |
| | | | | US | 2021024502 | A1 | 28 January 2021 |
| | | | | US | 11014913 | B2 | 25 May 2021 |
| | | | | PH | 12020551760 | A1 | 28 June 2021 |
| | | | | PL | 3788040 | T3 | 07 August 2023 |
| | | | | DK | 3788040 | T3 | 08 May 2023 |
| | | | | CR | 20200518 | A | 22 March 2021 |
| | | | | HRP | 20230458 | T1 | 21 July 2023 |
| | | | | LT | 3788040 | T | 26 June 2023 |
| | | | | PE | 20211382 | A1 | 27 July 2021 |
| | | | | CO | 2020013599 | A2 | 21 December 2020 |
| | | | | MX | 2020011465 | A | 07 December 2020 |
| | | | | US | 2020123134 | A1 | 23 April 2020 |
| | | | | US | 10870641 | B2 | 22 December 2020 |
| | | | | EA | 202092590 | A1 | 08 April 2021 |
| | | | | WO | 2019212937 | A1 | 07 November 2019 |
| | | | | JP | 2022017221 | A | 25 January 2022 |
| | | | | CA | 3098585 | A1 | 07 November 2019 |
| | | | | EP | 4234551 | A2 | 30 August 2023 |
| | | | | EP | 4234551 | A3 | 11 October 2023 |
| | | | | CL | 2020002821 | A1 | 19 February 2021 |
| | | | | TW | 202014416 | A | 16 April 2020 |
| | | | | AU | 2019262927 | A1 | 12 November 2020 |
| CN | 112812113 | A | 18 May 2021 | WO | 2021093817 | A1 | 20 May 2021 |
| | | | | CN | 112812113 | B | 20 September 2022 |
| CN | 113906031 | A | 07 January 2022 | AU | 2020285314 | A1 | 25 November 2021 |
| | | | | BR | 112021022960 | A2 | 04 January 2022 |
| | | | | JP | 2022535216 | A | 05 August 2022 |
| | | | | CA | 3136519 | A1 | 03 December 2020 |
| | | | | EP | 3976619 | A1 | 06 April 2022 |

Form PCT/ISA/210 (patent family annex) (July 2022)

**INTERNATIONAL SEARCH REPORT**
**Information on patent family members**

International application No.
**PCT/CN2023/110230**

| Patent document cited in search report | Publication date (day/month/year) | Patent family member(s) | Publication date (day/month/year) |
|---|---|---|---|
| | | US 2022153754 A1 | 19 May 2022 |
| | | WO 2020239864 A1 | 03 December 2020 |
| | | MX 2021014580 A | 11 January 2022 |
| | | KR 20220015426 A | 08 February 2022 |
| US 2011230472 A1 | 22 September 2011 | EP 2327704 A1 | 01 June 2011 |
| | | EP 2327704 A4 | 09 May 2012 |
| | | JPWO 2010024258 A1 | 26 January 2012 |
| | | WO 2010024258 A1 | 04 March 2010 |
| | | TW 201018691 A | 16 May 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2022094124 W